# EUROPÄISCHE PATENTSCHRIFT

(11) **EP 2 392 644 B1**
(45) Veröffentlichungstag und Bekanntmachung des Hinweises auf die Patenterteilung: **28.01.2015**
(21) Anmeldenummer: 11004178.7
(22) Anmeldetag: 20.05.2011
(51) Int. Cl.: G01N 33/50, C12N 5/00

(54) **In vitro-Testsystem für virale Infektionen**
In vitro test system for viral infections
Système de test in vitro pour infections virales

(30) Priorität: 02.06.2010 DE 102010023156
(43) Veröffentlichungstag der Anmeldung: 07.12.2011
(73) Patentinhaber: Fraunhofer-Gesellschaft zur Förderung der angewandten Forschung e.V., 80686 München (DE)
(72) Erfinder: Burger-Kentischer, Anke, Dr., 70569 Stuttgart (DE); Hogk, Ina, 71332 Waiblingen (DE); Finkelmeier, Doris, 70734 Fellbach (DE); Walles, Heike, Prof., Dr., 71063 Sindelfingen (DE); Rupp, Steffen, Dr. rer. nat., 70569 Stuttgart (DE); Kaufmann, Michaela, Dr., 58332 Schwelm (DE)
(74) Vertreter: Schwahn, Hartmut

(56) Entgegenhaltungen:
- WO-A1-2009/105130
- WO-A2-01/92476
- US-A- 5 755 814
- US-A- 5 955 343
- UROUKOV I S ET AL: "Electrophysiological measurements in three-dimensional in vivo-mimetic organotypic cell cultures: Preliminary studies with hen embryo brain spheroids", NEUROSCIENCE LETTERS, LIMERICK, IE, Bd. 404, Nr. 1-2, 14. August 2006 (2006-08-14), Seiten 33-38, XP027885797, ISSN: 0304-3940 [gefunden am 2006-08-14]
- SU YH ET AL: "Human Corneal Cells and Other Fibroblasts Can Stimulate the Appearance of Herpes Simplex Virus from Quiescently Infected PC12 Cells", JOURNAL OF VIROLOGY, vol. 73, no. 5, May 1999 (1999-05), pages 4171-4180,

## Beschreibung

Die Erfindung betrifft zellbasierte in vitro Assays und Testsysteme, besonders zur Untersuchung viraler Infektionen und antiviral wirkender Wirkstoffe. Sie stellt ein mehrschichtiges biologisches Gewebe als in vitro-Testsystem für Virusinfektionen und antiviral wirkende Substanzen bereit. Die Erfindung stellt auch Verfahren und Mittel zum Auffinden eines antiviralen Wirkstoffs in einem in vitro-Ansatz bereit.

Durch Viren werden eine Vielzahl von Infektionskrankheiten des Menschen oder von Tieren verursacht. Viren haben keinen eigenen Stoffwechsel, was die kausale Behandlung von viralen Infektionskrankheiten erschwert. Sie sind insbesondere durch Antibiotika nicht zu behandeln. Viruzide, also Virus abtötende Arzneimittel, gibt es derzeit nicht. Häufig ist das Immunsystem eines Patienten alleine nicht zur Eradikation des infizierenden Virus in der Lage. Bekannte antiviral wirkende Substanzen, so genannte Virostatika, verhindern die Ausbreitung oder Vermehrung der Viren durch verschiedene Wirkmechanismen, die im Zusammenhang mit dem Infektionszyklus oder Vermehrungszyklus der Viren stehen. Bekannte Virustatika wirken besonders als DNA-Polymeraseinhibitoren. Bekannte nucleotidisch wirkende Substanzen sind Idoxuridin, Aciclovir und Ganciclovir und deren Derivate. Andere Polymeraseinhibitoren sind beispielsweise Poscarnet und Ribavirin.

Infektionen mit dem Herpes simplex Virus (HSV) gehören zu den häufigsten Erkrankungen des Menschen. Mehr als 90 % der gesamten Weltbevölkerung sind mit diesem Virus infiziert. Herpes simplex Viren (HSV) werden in zwei eng verwandte Virusspezies eingeteilt (humanes Herpesvirus 1 (HSV1) und humanes Herpesvirus 2 (HSV2). Die Herpesviren verursachen sehr verschiedene Erkrankungen des Herpes simplex, darunter auch Herpes simplex-Enzevalitis und der Herpes des Neugeborenen. Am häufigsten sind Herpes labialis und Herpes genitalis.

Charakteristisch für HSV ist die Persistenz. Nach erfolgter Erstinfektion der Zellen des tierischen oder menschlichen Wirtsorganismus, besonders von Epithelzellen der Schleimhaut, kommt es zu einer Ausbreitung des Virus in neuronale Zellen, besonders Zellen sensorischer Neurone, welche die primär infizierte Region innervieren. Über retrograden axonalen Transport gelangen die Viren in die Ganglien und treten dort typischerweise in einen Zustand der Latenz. Die Virus-DNA persistiert, im Wesentlichen unerkannt vom Immunsystem des Wirtes, als zirkuläres Episom im Zellkern der Ganglien. Während der Latenzphase,findet keine Virusreplikation statt, der infizierte Wirt ist symptomfrei. Durch Stressoren, wie geschwächtes Immunsystem, Sonnenlichtexposition, entzündliche Vorgänge, hormonelle oder psychologische Einflüsse (neuroendrokrinologische Konditionen) oder Nervenreizung, wird eine Reaktivierung der latenten Viren ausgelöst. Dabei wandern Virionen aus den Ganglien axonal zurück in die Peripherie und reinfizieren dort das Gewebe, besonders die Eptihelzellen. Durch einen lytischen Replikationszyklus im Epithelgewebe und die daraus resultierende Gewebezerstörung zeigt sich das typische Krankheitsbild einer Herpesinfektion. Die bekannte Therapie einer Herpesinfektion ist unzureichend. Bekannte Virustatika können lediglich die Symptome lindern und die Infektionszeit verkürzen, vermögen jedoch nicht die Persistenz der Viren in den Gangienzellen zu beenden (Eradikation).

Viele der bislang durchgeführten Untersuchungen zu den Latenzmechanismen und Reaktivierungsanalysen werden mit Hilfe von mamalischen Zelllinien gewonnen. Diesen Testsystemen fehlt jedoch die unmittelbare Übertragbarkeit auf die in vivo-Situation im Patienten. Folglich werden bei Untersuchungen der Infektions- und Latenzmechanismen, besonders aber bei der Wirkstoffentwicklung weiterhin Tierversuche eingesetzt. Es ist wünschenswert, in vitro-Testsysteme zu entwickeln, die eine unmittelbare Übertragbarkeit der damit gefundenen Ergebnisse auf die Situation im infizierten Patienten erlauben und Tierversuche ablösen können.

Mit Hilfe standardisierter dreidimensionaler in vitro-Hautäquivalente, die aus primären Hautzellen und/oder Hautzelllinien neu konstituiert werden, kann menschliche Haut reproduzierbar nachgebildet werden. Physiologisch sind solche Hautäquivalente weitgehend mit nativer Haut vergleichbar. Sie dienen bekanntermaßen als in vitro-Testsysteme für Hautgewebe (in vitro-Hautmodell).

Aus der WO 01/92476 A2 ist ein mehrschichtiges in vitro-Hautgewebemodell bekannt, welches eine Kollagenbiomatrix mit darin eingebetten Fibroblasten und eine Schicht enthaltend Keratinozyten aufweist. Dieses kann mit einem pathogenen oder parasitären Mikroorganismen kultiviert werden. Aus Ying-Hsiu et al., Journal of Virology, 73(5), 1999, p. 4171-4180, sind HSV-1 latent infizierte neuronale PC-12-Zellen bekannt.

Das der Erfindung zugrundeliegende technische Problem bestand darin, ein in vitro-Testsystem bereitzustellen, mittels dessen besonders Latenzmechanismen und Reaktivierungsanalysen bei Virusinfektionen, besonders bei HSV-Infektionen, durchgeführt werden können und deren Ergebnisse besonders direkt auf die in vivo-Situation in einem menschlichen oder tierischen Organismus übertragbar sind. Besonders soll das in vitro-Testsystem ein Wirkstoffscreening zum Auffinden antiviral aktiver Substanzen ermöglichen.

onszeit verkürzen, vermögen jedoch nicht die Persistenz der Viren in den Gangienzellen zu beenden (Eradikation).

Viele der bislang durchgeführten Untersuchungen zu den Latenzmechanismen und Reaktivierungsanalysen werden mit Hilfe von mamalischen Zelllinien gewonnen. Diesen Testsystemen fehlt jedoch die unmittelbare Übertragbarkeit auf die in vivo-Situation im Patienten. Folglich werden bei Untersuchungen der Infektions- und Latenzmechanismen, besonders aber bei der Wirkstoffentwicklung weiterhin Tierversuche eingesetzt. Es ist wünschenswert, in vitro-Testsysteme zu entwickeln, die eine unmittelbare Übertragbarkeit der damit gefundenen Ergebnisse auf die Situation im infizierten Patienten erlauben und Tierversuche ablösen können.

Mit Hilfe standardisierter dreidimensionaler in vitro-Hautäquivalente, die aus primären Hautzellen und/oder Hautzelllinien neu konstituiert werden, kann menschliche Haut reproduzierbar nachgebildet werden. Physiologisch sind solche Hautäquivalente weitgehend mit nativer Haut vergleichbar. Sie dienen bekanntermaßen als in vitro-Testsysteme für Hautgewebe (in vitro-Hautmodell).

Das der Erfindung zugrundeliegende technische Problem bestand darin, ein in vitro-Testsystem bereitzustellen, mittels dessen besonders Latenzmechanismen und Reaktivierungsanalysen bei Virusinfektionen, besonders bei HSV-Infektionen, durchgeführt werden können und deren Ergebnisse besonders direkt auf die in vivo-Situation in einem menschlichen oder tierischen Organismus übertragbar sind. Besonders soll das in vitro-Testsystem ein Wirkstoffscreening zum Auffinden antiviral aktiver Substanzen ermöglichen.

Das technische Problem wird vollständig gelöst durch die Bereitstellung eines mehrschichtigen biologischen in vitro-Gewebes, das eine Dermisschicht und bevorzugt auch eine Epidermisschicht enthält, wobei die Dermisschicht im Wesentlichen aus einer Kollagenbiomatrix mit darin eingebetteten, das heißt integrierten, Fibroblasten aufgebaut ist oder diese enthält. Die darauf angeordnete Epidermisschicht enthält im Wesentlichen Epithelzellen, das heißt besonders Keratinozyten. Dermisschicht und Epithelschicht können ein Modellsystem bilden, das menschlichem Hautgewebe ähnlich oder äquivalent ist. Das erfindungsgemäße in vitro-Testmodell basiert demgemäß auf einem in vitro-Hautmodell. Erfindungsgemäß ist dieses mehrschichtige biologische Gewebe vor allem dadurch gekennzeichnet, dass zumindest in dessen Dermisschicht, zusätzlich zu den Fibroblasten, latent infizierte, neuronale Zellen integriert sind.

In einer besonderen Ausgestaltung ist die virale Infektion der neuronalen Zellen eine aktive oder reaktivierbare latente Infektion mit dem Herpes simplex Virus (HSV), besonders mit dem Herpes simplex Virus Typ 1 (HSV1). Die Erfindung ist aber nicht auf diesen Virustyp beschränkt. In anderen bevorzugten Ausgestaltungen des in vitro-Testsystems ist der Virus ausgewählt aus: CMV, VZV, HBV, Influenza A, Influenza B, RSV und HCV.

In einer besonderen Ausgestaltung sind die in das in vitro-Testsystem eingebetteten neuronalen Zellen die Zelllinie von Phäochromocytomzellen; besonders sind dies Zellen vom Typ PC12 (Phaeochromocytoma 12).

Die Erfindung sieht also vor allem vor, in ein mehrschichtiges, so genanntes dreidimensionales in vitro-Hautäquivalent latent viral infizierte neuronale Zellen zu integrieren, um ein neuartiges in vitro-Testsystem zu erhalten. Damit können vor allem der Verlauf und die Mechanismen einer viralen Infektion, das heißt besonders die Reaktivierung einer latenten Virusinfektion sowie die Interaktion der infizierten neuronalen Zellen mit den Hautzellen, untersucht werden. Dies erlaubt gegenüber bekannten Testsystemen die einfachere, zuverlässigere und auch aussagekräftigere Durchführung von solchen Untersuchungen, besonders aber von Screenings zum Zwecke des Auffindens und Charakterisierens potentiell antiviral aktiver Substanzen.

In einer besonderen Ausgestaltung sind in der Epidermisschicht des in vitro-Testsystems die Epithelzellen ausgewählt aus Zellen von Keratinozytenzelllinien und/oder primären Keratinozyten. In einer besonderen Variante davon entstammen die Epithelzellen der Keratinozytenzelllinie HaCaT (human adult low calcium high temperature) oder sind davon abgeleitete Zellen. Besonders HaCaT-Zellen zeigen überraschenderweise ein dem in vivo-Zustand- eines Hautgewebes weitgehend entsprechendes Differenzierungsverhalten und können so die Übertragbarkeit der mittels des erfindungsgemäßen in vitro-Testsystems gefundenen Erkenntnisse auf die in vivo-Situation verbessern. Besonders im Zusammenhang mit einer überwiegend oder ausschließlich aus HaCaT-Zellen oder davon abgeleiteten Zellen aufgebauten Epidermisschicht ist vorgesehen, dass das in vitro-Gewebe in Kulturmedium kultiviert wird, das zumindest einen oder mehrere der Kulturfaktoren, ausgewählt aus: TGF-α, GM-CSF und IL-1α, enthält. Solche Kulturfaktoren sind geeignet, die Angleichung des Zellstatus an die in vivo-Situation nochmals zu verbessern.

In einer besonderen Variante sind die zum Aufbau der Epidermisschicht eingesetzten Keratinozyten, alternativ oder zusätzlich, genetisch modifiziert: Zum Zwecke der Untersuchung der Signalwege bei einer Virusinfektion sind die Keratinozyten bevorzugt für einen oder mehrere Pattern Recognition Receptoren (PRR) defizient, das heißt sie stellen für solche Rezeptoren eine knock down-Mutante oder gegebenenfalls eine knock out-Mutante dar. In einer besonderen Variante ist der defiziente PRR ausgewählt aus der Gruppe der Toll-like Rezeptoren (TLR): bevorzugt sind TLR2, TLR5 und TLR9. Demgemäß stellt die Erfindung in einer besonderen Ausgestaltung ein in vitro-Testsystem bereit, worin die Epidermisschicht Keratinozyten enthält oder aus dieser aufgebaut ist, die knock down-Mutanten oder knock out-Mutanten sind für eine oder mehrere TLR, besonders für TLR2, TLR5 und/oder TLR9. Um dies zu erreichen, können besonders HaCaT-Zellen mit small interfering RNA (siRNA) für TLR codierenden Plasmiden in an sich bekannter Weise transfiziert werden (Nucleofektion, RNA-interference (RNAi)-Verfahren). Durch siRNA können in an sich bekannter Weise zelluläre Proteine unterdrückt, das heißt "herabgeregelt" werden. Eine vollständige Unterdrückung der Proteine braucht nicht erreicht werden; die Erfindung erfordert in dieser Ausgestaltung eine vollständige Unterdrückung (knock out) nicht. Die Erfindung ist nicht auf diese Ausführung zur Erzeugung einer knock down-Mutante beschränkt. Bevorzugt sind die knock down-Mutanten HaCaTΔTLR2, HaCaTΔTLR5 und HaCaTΔTLR9 und knock down-Kombinationen (Doppel-, Mehrfachmutanten) davon. Die Erfindung ist nicht auf diese knock down-Mutanten beschränkt.

In einer alternativen Variante enthält die Epidermisschicht, zusätzlich oder ausschließlich, primäre Epithelzellen, die insbesondere in vorangehenden Schritten gewonnen, aus Gewebe der Mundschleimhaut oder dazu vergleichbarer Gewebe aus Darm, Magenschleimhaut, Haut, Cornea, Trachea und anderen Epithelgeweben. Eine besondere Quelle von Epithelzellen stellen menschliche Spendergewebe, besonders Vorhautgewebe (Präputium) dar.

In einer besonderen Ausgestaltung ist die Dermisschicht des in vitro-Testgewebes aus einer Kollagenbiomatrix aufgebaut, die im Wesentlichen Kollagen vom Typ I enthält oder bevorzugt daraus besteht. Kollagen Typ I wird in einer bevorzugten Variante als überwiegend oder im Wesentlichen natives Kollagen bereitgestellt. Dazu wird besonders das Kollagen möglichst frisch und ohne denaturierende Zwischenschritte aus kollagenhaltigem Gewebe, besonders aus an Kollagen Typ I reichem Gewebe, beispielsweise Rattenschwanzsehnen, schwach essigsauer oder alternativ mittels Harnstoff extrahiert und vorzugsweise durch Anhebung des pH-Werts und/oder durch Renaturieren in Puffer, besonders durch Zumischen einer gepufferten Zellsuspension, zum Gelieren gebracht, um eine Kollagenbiomatrix, besonders mit darin eingebetteten Zellen, besonders Fibroblasten, bevorzugt mit erfindungsgemäß zusätzlich eingebetteten nuronalen Zellen, zu erhalten, die die Dermisschicht bildet. Bei der Herstellung/Bereitstellung der Kollagenbiomatrix werden vorzugsweise denaturierende Schritte wie Salzfällung, starke Alkalität oder Acidität, thermische Denaturierung sowie Lyophilisierung vermieden oder gänzlich ausgeschlossen. Um eine native Kollagenstruktur zu erhalten, wird das kollagenhaltige Ausgangsgewebe bevorzugt bei geringer Säurekonzentration, besonders pH 4 oder mehr, für eine vergleichsweise lange Zeit, besonders 3 bis 4 Tage, in der Kälte extrahiert und durch Verdünnung mit Pufferlösung und/oder durch Anheben der Temperatur, zum Beispiel bei Raumtemperatur, geliert.

In einer besonderen Variante enthält die Dermisschicht primäre Fibroblasten, bevorzugt humane primäre Fibroblasten. Diese werden vorzugsweise frisch aus humanem Spendergewebe, beispielsweise Vorhautgewebe, gewonnen. Dazu werden in einem ersten Schritt in dem Spendergewebe die Hautschichten getrennt, und aus der isolierten Dermisschicht des Spendergewebes vereinzelte Fibroblasten werden rekultiviert und in die Kollagenbiomatrix eingebettet, um eine neu konstituierte Dermisschicht zu bilden.

In einer weiteren besonderen Ausgestaltung sind in das erfindungsgemäße Testgewebe zusätzlich immunkompetente Zellen, das heißt besonders Immunzellen, integriert. Dadurch kann die Aussagefähigkeit des auf dem erfindungsgemäßen Gewebe basierenden in vitro-Testsystems verbessert werden. Bevorzugt sind hierbei besonders noch inaktive epidermal gelegene dendritische Zellen, besonders bevorzugt die Langerhanszellen. Die immunkompetenten Zellen dienen besonders der Unterstützung der interzellulären Kommunikation in dem Testgewebe, indem sie Antigene prozessieren und gegebenenfalls weiteren Immunzellen präsentieren können. Die Erfindung sieht dabei vor, dass die immunkompetenten Zellen auf oder in die Dermisschicht ausgesät oder zusammen mit den Fibroblasten in diese eingebettet werden.

Gegenstand der Erfindung ist auch ein Verfahren zur Herstellung eines mehrschichtigen biologischen Gewebes, das besonders als in vitro-Testsystem, besonders zur Untersuchung von Virusinfektionen und/oder zum Screening antiviral wirkender Substanzen, eingesetzt werden kann. In einem ersten Verfahrensschritt wird zur Herstellung eines mehrschichtigen Basisgewebes eine Kollagenbiomatrix bereitgestellt. Diese wird bevorzugt wie hierin beschrieben hergestellt. Bevorzugt enthält die Kollagenbiomatrix im Wesentlichen Kollagen Typ l in nicht denaturierter Form oder besteht daraus. In die Biomatrix werden erfindungsgemäß die hierin an anderer Stelle näher charakterisierten Fibroblastenzellen eingebettet. Dazu werden die Fibroblasten in der noch nicht ausgehärteten gelierten Kollagenlösung suspendiert: Alternativ wird die Kollagenlösung mit einer Suspension von Fibroblasten in Puffer und/oder Zellkulturmedium vermischt. Die Kollagenbiomatrix härtet mit den darin suspendierten Fibroblasten aus.

Erfindungsgemäß ist vorgesehen, dass in die Dermisschicht zusätzlich latent viral infizierte neuronale Zellen, wie sie hierin an anderer Stelle näher charakterisiert sind, eingebettet werden. Die Einbettung der neuronalen Zellen erfolgt bevorzugt zusammen mit dem Einbetten der Fibroblasten. Dazu werden sowohl Fibroblasten als auch die latent viral infizierten neuronalen Zellen suspendiert, und die Kollagenlösung härtet zusammen mit den Zellen zu einer Kollagenbiomatrix mit darin eingebetteten Fibroblasten und neuronalen Zellen aus. Diese Kollagenbiomatrix mit den darin eingebetteten Zellen bildet so die Dermisschicht des erfindungsgemäßen in vitro-Testgewebes.

Weiter wird auf der Dermisschicht zusätzlich eine epidermale Schicht aufgebaut. Dies erfolgt besonders durch Überschichten der Dermis mit Keratinozyten, die hierin an anderer Stelle näher charakterisiert werden. Dabei ist besonders vorgesehen, dass die dermale Kollagenmatrix vor dem Aufbringen der Keratinozyten zunächst mit Fibronektin oder einer ähnlichen Komponente überschichtet wird, welche die Basalmembran zwischen der Dermis und der darauf platzierten epidermalen Schicht bilden kann. So wird ein mehrschichtiges biologisches in vitro-Testgewebe, enthaltend Fibroblasten und latent viral infizierte neuronale Zellen sowie Epithelzellen, das heißt Keratinozyten erhalten.

In einer besonderen Ausgestaltung des Verfahrens werden die neuronalen Zellen vor dem Einbetten in das Testgewebe durch Infektion mit einem Virus, in der vorliegend näher beschriebenen Erfindungsvariante mit dem Herpes simplex Virus vom Typ 1 (HSV1), infiziert. Dies kann in an sich bekannter Weise erfolgen. Es wird dabei eine latente und reaktivierbare Virusinfektion in den neuronalen Zellen erhalten. Das in vitro-Testmodell erhält so in dieser erfindungsgemäßen Ausgestaltung eine latenzausbildende neuronale Komponente.

Die latente Infektion kann durch molekularbiologische Analyse verifiziert werden. In einer bevorzugten Variante wird dazu eine quantitative RT-PCR durchgeführt. Dabei wird vorzugsweise spezifisch auf latenzassoziierte Transkripte (LAT-Gene) regulierende miRNA hin untersucht. Es versteht sich, dass der Nachweis der vorgesehenen latenten Virusinfektion nicht auf diese Verfahren beschränkt ist.

Das in vitro-Testgewebe wird zunächst bevorzugt in Submerskultur in an sich bekannter Weise kultiviert. Diese Kultivierungsphase dauert etwa 6 Tage. Nach mechanischer Stabilisierung und/oder Abschluss erster Phasen des Aufbaus intakter Gewebeschichten durch die eingebrachten Zellen folgt im Anschluss eine Kultivierung in der so genannten Airliftkultur (airlift-Phase). Die Airliftphase dauert etwa 14 bis 15 Tage. Während der airlift-Phase wird vorzugsweise die Hornifizierung der Keratinozyten (Epidermis) gefördert, besonders mittels Calcium-reichem Medium.

Die Erfindung sieht zur Herstellung des in vitro-Testgewebes und zu dessen erfindungsgemäße Verwendung, besonders im Zusammenhang mit dem Wirkstoffscreening vor, dass eine spezifische Reaktivierung der Virusinfektion in den latent viral infizierten neuronalen Zellen erfolgt. In besonderer Ausgestaltung erfolgt dies durch mindestens einmalige, bevorzugt zweimalige, Exposition mit mindestens einen reaktivierenden Stressor. Bevorzugt ist dieser Stressor ausgewählt aus energiereicher elektromagnetischer Strahlung, besonders UVB-Strahlung (UVB-Exposition), und/oder thermischer Einwirkung (Hitzeexposition). Die Möglichkeiten der Reaktivierung der Virusinfektion sind nicht auf diese Stressoren beschränkt. Weitere Stressoren, die im Sinne der Erfindung alternativ oder zusätzlich zur Virusreaktivierung eingesetzt werden können, sind chemische Agenzien, zelluläre Botenstoffe, besonder Neurotransmitter und Neuroindikatoren, Änderungen in der lonenzusammensetzung des Mediums, besonders depolarisierende Ionenänderungen, pH-Wert-Änderung undr die Verminderung des Sauerstoffpartialdrucks bei der Kultivierung.

Bevorzugt liegt zwischen einer ersten und einer zweiten Exposition ein Zeitraum von etwa 24 Stunden. Besonders liegt der Zeitpunkt der abschließenden (zum Beispiel zweiten) Exposition zeitlich im Bereich von mindestens 7 Stunden und besonders bis etwa 25 Stunden vor dem Abschluss der Kultivierungsphase, das heißt vor Fixierung des Testgewebes. In einer besonderen Variante findet zur Reaktivierung eine Exposition des Gewebes mit UVB-Licht (312 nm) mit etwa 1500 mJ bevorzugt zweimal für jeweils 6 bis 10 min statt. Die erste Exposition erfolgt besonders von 28 bis 34 Std. vor Fixierung, die zweite Exposition erfolgt besonders von 5 bis 9 Std. vor Fixierung.

Gegenstand der Erfindung ist auch ein Verfahren zum Auffinden (Screening) eines antiviral wirkenden Wirkstoffs und zu dessen Auswahl aus einer Gruppe von zu untersuchenden Substanzen. In einem ersten Schritt wird das erfindungsgemäße in vitro-Testgewebe mit darin integrierten latent viral infizierten neuronalen Zellen bereitgestellt. In einem weiteren Schritt wird die latente Virusinfektion der neuronalen Zellen spezifisch reaktiviert.

Das in vitro-Testgewebe wird in einem weiteren Schritt mit der auf antivirale Eigenschaft zu untersuchenden Substanz (potentieller Wirkstoff) in Kontakt gebracht. Das Inkontaktbringen findet in einer ersten Variante zeitlich noch vor der spezifischen Virusreaktivierung statt. In einer alternativen Variante findet dieses Inkontaktbringen zeitlich erst nach spezifischer Virusreaktivierung statt.

In einem weiteren Schritt wird der Umfang der Virusaktivierung untersucht, dabei zeigt besonders die Reduzierung des Umfangs der Virusaktivierung nach Inkontaktbringen des in vitro-Testgewebes mit der zu untersuchenden Substanz im Vergleich zu der Virusinaktivierung einen parallelen Kontrollansatz, welcher nicht mit der zu untersuchenden Substanz in Kontakt gebracht wurde, eine antivirale Wirkung der Substanz an. Alternativ kann die Reduzierung der Viruslast durch die untersuchende Substanz mit einem Kontrollansatz verglichen werden, worin ein bekannter antiviral wirkender Wirkstoff als Vergleichswirkstoff eingesetzt wurde. Als Kontrolle können bekannte Wirkstoffe, beispielsweise Virostatika wie Aciclovir, appliziert werden.

In einem nachfolgenden Schritt wird die so als antiviral wirkend charakterisierbare Substanz identifiziert und/oder aus der Gruppe der zu untersuchenden Substanzen gegebenenfalls automatisiert ausgewählt und bereitgestellt.

Die zu untersuchende Substanz wird in einer ersten Variante der Erfindung topikal auf das in vitro-Testgewebe aufgebracht. Durch topikale Applikation können besonders an der Hautoberfläche therapeutisch nutzbare Wirkstoffe reproduzierbar und unter standardisierten Bedingungen in vitro getestet oder identifiziert werden. In einer alternativen Variante wird die zu untersuchende Substanz in das das Testgewebe umgebende Kulturmedium appliziert.

In einer Variante wird die zu untersuchende Substanz zeitlich im Anschluss an die erfolgte Reaktivierung des Virus mit dem in vitro-Testgewebe in Kontakt gebracht, und zwar bevorzugt in einem Zeitraum von 2 bis 7 Tagen, bevorzugt von 2 bis 4 Tagen, nach Virusreaktivierung. In einer alternativen Variante erfolgt das Inkontaktbringen des Testgewebes mit der zu untersuchenden Substanz bereits zeitlich vor oder, alternativ oder zusätzlich, im Verlauf der Induzierung der Reaktivierung. Die Zugabe der Substanz kann bereits ab Tag 1 der Kultivierungsphase des Testgewebes in der Airliftkultur erfolgen.

Zum Nachweis des Umfangs der Virusaktivierung sieht die Erfindung in einem ersten Ansatz vor, eine quantitative RT-PCR durchzuführen. Dabei werden spezifisch virusrelevante Genexpressionen in an sich bekannter Weise untersucht. In einem alternativen Ansatz wird eine immunhistochemische Färbung mit insbesondere HSV1 spezifischen Antikörpern durchgeführt. Dies sind in besonderer Ausgestaltung polyklonale anti-HSV1 Antikörper. Die immunhistochemische Färbung wird in an sich bekannter Weise durchgeführt und kann gegebenenfalls in an sich bekannter Weise quantifiziert werden. Werden dabei beispielsweise die Testgewebe in Multiwellplatten kultiviert, kann die Färbung unmittelbar im Testgewebepräparat mikroskopisch und/oder densitometrisch automatisiert quantifiziert werden.

Die Erfindung wird durch die nachfolgenden Beispiele und Figuren näher charakterisiert, ohne dass diese beschränkend zu verstehen wären.

Die Figuren zeigen:

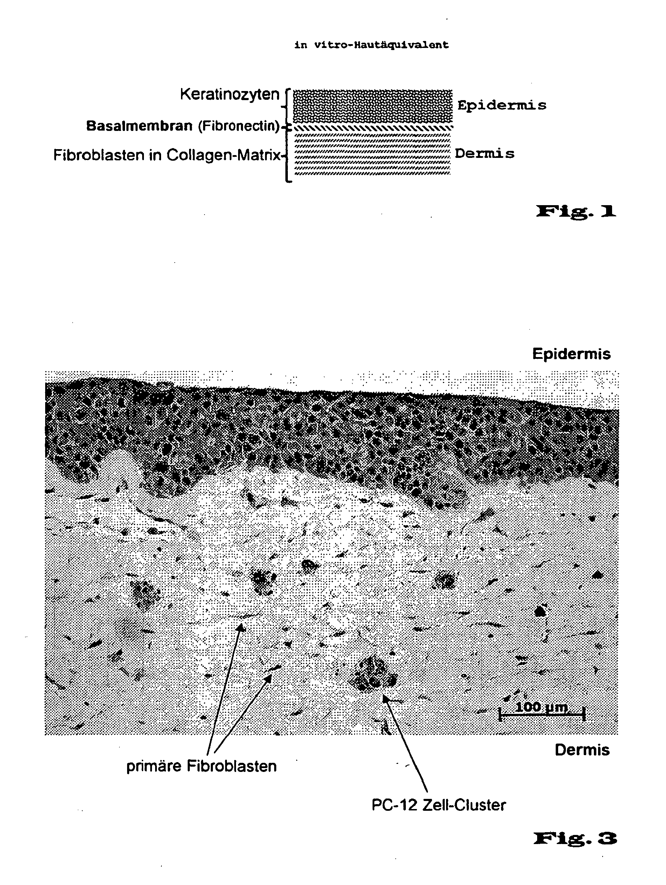

Figur 1: Schematische Darstellung der Struktur des dreidimensionalen in vitro-Hautäquivalents, bestehend aus einer Epidermisschicht mit Keratinozyten und einer Dermisschicht mit in einer Kollagenbiomatrix eingebetteten Fibroblasten. Zwischen Epidermis und Dermis bildet sich eine Basalmembran (Fibronektin) aus.

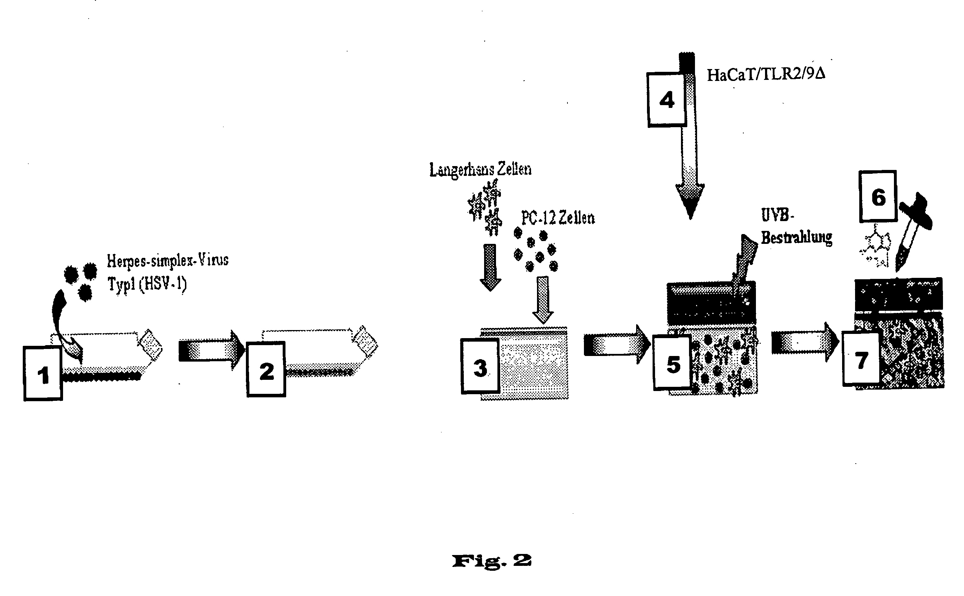

Figur 2: Spezifische Ausgestaltung des Verfahrens zur Herstellung eines in vitro-Testsystems zur Untersuchung von viralen Infektionen und für Wirkstoffscreenings: In einem ersten Schritt (1) wird eine neuronale Zelllinie (PC-12) mit HSV-1 infiziert; es bildet sich eine latente Infektion aus (2). Die latent infizierten PC-12-Zellen werden, gegebenenfalls zusammen mit immunkompetenten Langerhans-Zellen, in die Dermisschicht integriert (3). Zum vollständigen Aufbau des 3D-Gewebes werden nach Aufbringen einer Fibronectinschicht immortalisierte modifizierte Keratinozyten-Zelllinie, beispielsweise HaCaT/ΔTLR2/ATLR9, auf die Dermisschixht appliziert, um eine Epidermisschicht zu bilden (4). Durch UVB-Bestrahlung (312 nm, 1500 mJ, 8 min, zum Beispiel zweimal im Abstand von 24 Stunden), erfolgt die spezifische Virusreaktivierung in den intePC-12-Zellen (5). Die infolge der Reaktivierung auftretende aktive Virusinfektion sowie die einsetzende interzelluläre Kommunikation kann durch Inkontaktbringen mit antiviralen Wirkstoffen modifiziert werden (6). Die Viruslast im Gewebe wird im Anschluss daran mittels histologischer Färbung bestimmt (7).
Figur 3: Histologischer Querschnitt eines erfindungsgemäßen in vitro-Testsystems (HE-Färbung) mit Dermisschicht (hell) und darüberliegender Epidemisschicht (dunkel). In der Dermisschicht sind primäre Fibroblasten und PC-12-Zellen (Cluster) eingebettet..

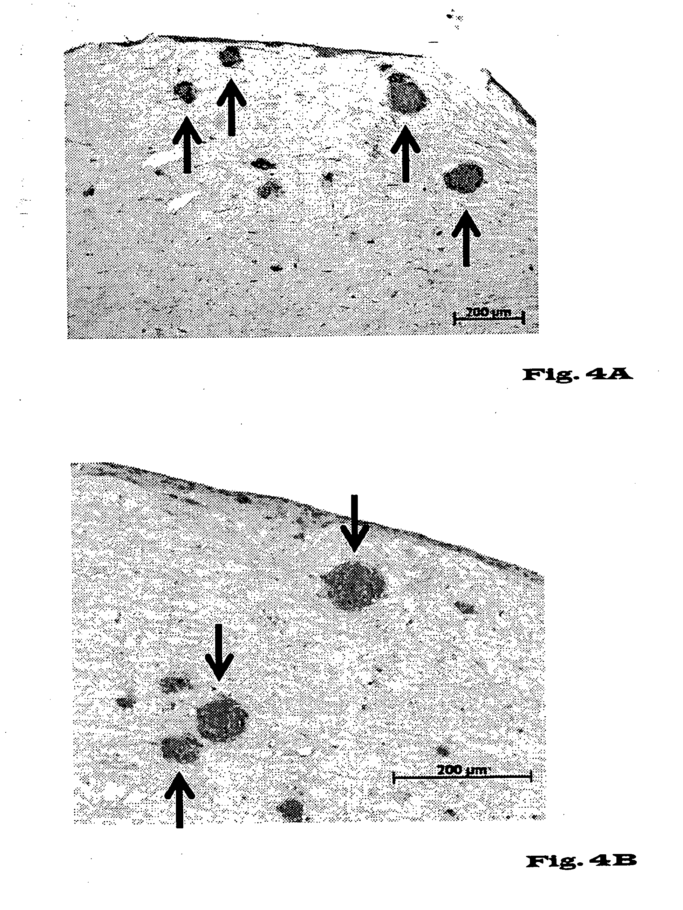

Figur 4AB: Antikörper-gefärbte Querschnitte der Hautmodelle nach Figur 3 zum spezifischen Nachweis von PC-12 Zellen (Figur 4B) sowie sowie die Isotypkontrolle (Figur 4A); Primär-Ak: Anti-Tyrosin hydroxylase (Fa. abcam), Verd. 1:400, sowie lgG2a Isotypkontrolle (Fa. Dako), Verd. 1:400; Sekundär-Ak: Anti-Mouse (Fa. Dako).

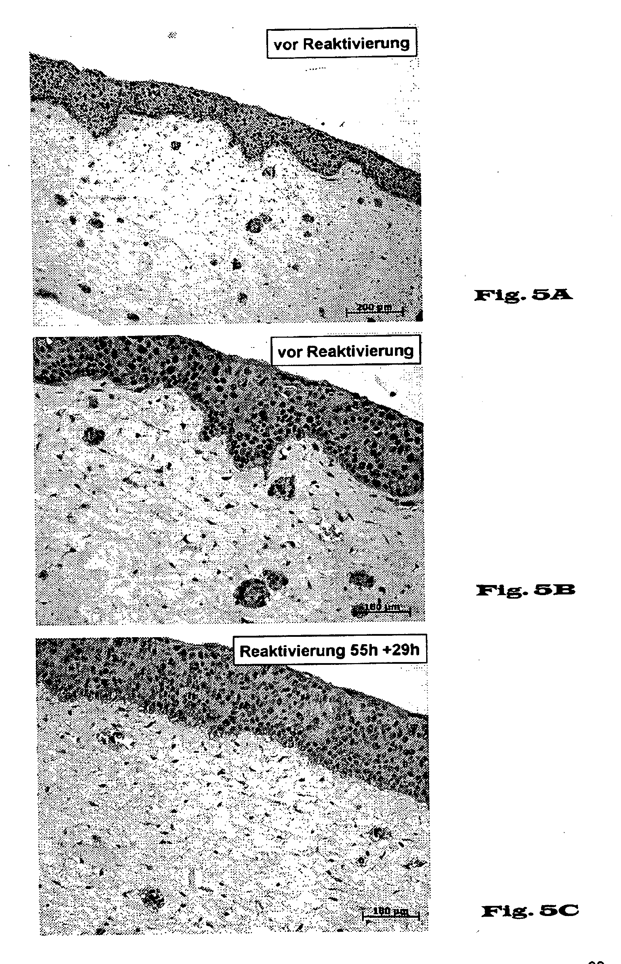

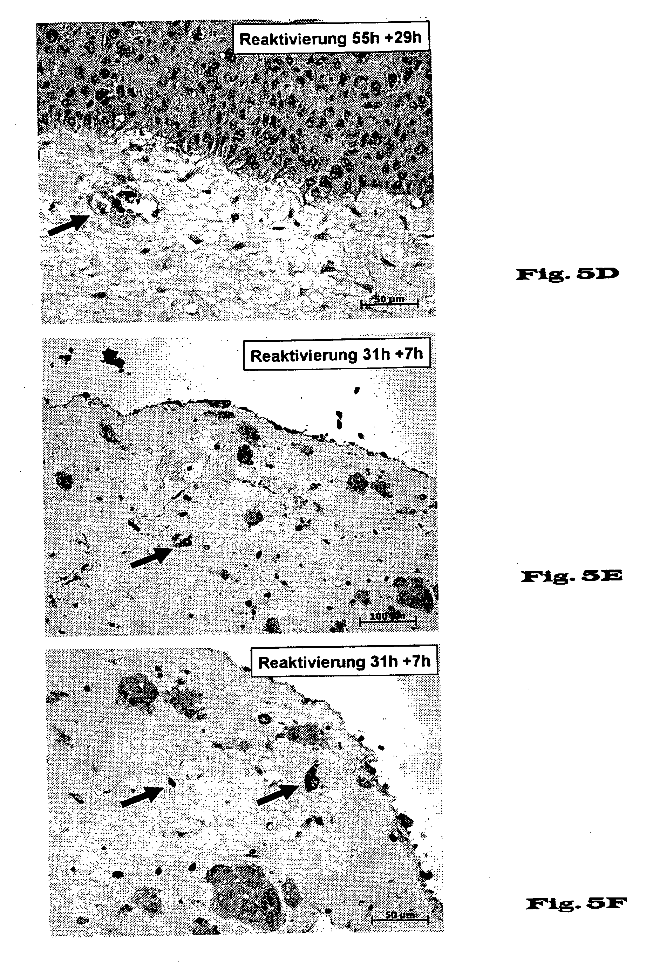

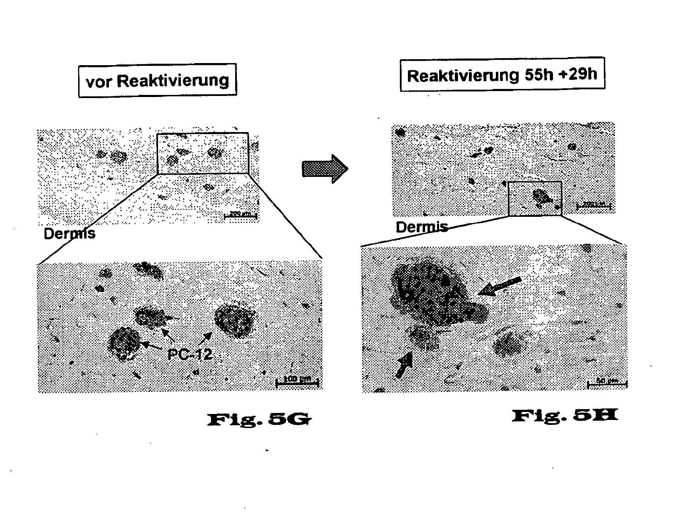

Figur 5ABCD: Antikörper-gefärbte Querschnitte der Hautmodelle nach Figur 3 zum spezifischen Nachweis von HSV-1. Vor (Figur 5A, Figur 5B und Figur 5G) sowie im Anschluss an eine zweimalige UVB-Exposition zur Aktivierung, und zwar 55 Std. und 29 Std. (Figur 5C, Figur 5D und Figur 5H) oder 31 Std und 7 Std. (Figur 5E und Figur 5F) vor Beendigung der Kultivierung; Primär-Ak: polyclonal Anti-HSV-1 (Fa. Biogenex), Verd.1:300; Sekundär-Ak: Anti-Mouse (Fa. Dako).

### Ausführungsbeispiel

Zur Infektion der neuronalen PC-12 Zelllinie werden die Zellen in eine Vertiefung einer 6-Well-Platte ausgesät und mit dem Herpes simplex Virusstamm HF (ATCC, VR260) bei einer Viruslast von 1 PFU/Zelle für zwei Stunden bei 37°C inkubiert. Anschließend werden alle nicht adsorbierten Virionen durch mehrmaliges Waschen mit Puffer (PBS) entfernt. Die Zellen werden für weiter 24 Stunden mit frischem Medium kultiviert.

Zur Ausbildung einer latenten HSV-1 Infektion werden die Zellen mehrfach subkultiviert (2 bis 4 Passagen). Vor der Integration in das in vitro-Testmodell wird geprüft, dass keine Virusaktivität mehr nachweisbar ist. Hierzu wird vor jeder Passage etwa 1 mm Kulturüberstand zurückgehalten und unter Verwendung eines zellbasierten Testassays mit Vero B-Zellen der Infizierungsgrad (TCID50) bestimmt. Bei dieser Methode der Endverdünnung wird festgestellt, in welcher Verdünnungsstufe des zu untersuchenden Materials auch eine Infektion stattfindet. Hierbei werden mehrere Verdünnungsstufen parallel angesetzt und ermittelt, bei welcher Verdünnung 50 % der beimpften Zellkulturen infiziert werden. Zum Nachweis der latenten Infektion wird alternativ die Methode der in situ-Hybridisierung herangezogen. Dabei werden latenzassoziierte Transkripte (LAT) nachgewiesen.

Der Aufbau des in vitro-Testgewebes erfolgt nach einem für die Einbettung der PC-12 Zellen optimierten Protokoll: Grundsätzlich erfolgt der Aufbau in zwei Schritten. In einem ersten Schritt wird der dermale Teil des Testgewebes aufgebaut, worin primäre Fibroblasten sowie latent mit HSV-1 infizierte PC-12 Zellen in eine Kollagenmatrix mit Kollagen vom Typ I integriert werden. Hierzu werden jeweils 0,25 x 10⁶ ml Fibroblasten sowie 0,14 x 10⁶ ml latent infizierte PC-12 Zellen luftblasenfrei in einer frisch hergestellten Lösung von Kollagen l resuspendiert und die Suspension in ein Insert einer 24-Well-Platte transferiert.

In einem zweiten Schritt folgt das Überschichten der Dermis mit humanen Keratinozyten (0,4 x 10⁶ pro ml), welche dann die epidermale Schicht bilden. Vor dem Aufbringen der Keratinozyten wird die dermale Kollagenmatrix mit Fibronektin überschichtet, welches dann die Basalmembran bildet. Als Negativkontrolle wird als Testgewebe ein Hautäquivalent mit nicht infizierten PC-12 Zellen aufgebaut.

In einem weiteren Ansatz wird eine Immunkomponente, besonders Langerhanszellen, in das Testmodell integriert: In einem ersten Ansatz davon werden die Immunzellen vor der Aussaat der Keratinozyten auf oder in die Biomatrix ausgesät, in einem weiteren Ansatz davon werden Immunzellen während oder anschließend an die Aussaat der Keratinozyten auf oder in die Biomatrix ausgesät.

Der Aufbau des in vitro-Testgewebes umfasst insgesamt etwa 21 Tage. Die Testgewebe durchlaufen in dieser Zeit unterschiedliche Kultivierungsphasen. In den ersten sechs Tagen, der so genannten Submers-Phase, werden die Gewebe vollständig mit Medium überschichtet kultiviert. Anschließend folgt eine 14- bis 15-tägige Airlift-Phase, worin die Testassays an dem Gewebe durchgeführt werden. Nach Abschluss der Kultivierungsphase werden die Testgewebe wahlweise in Bouin'scher Lösung oder mittels Histofix® in an sich bekannter Weise fixiert und anschließend bevorzugt in Paraffin eingebettet. In an sich bekannter Weise werden Gewebeschnitte hergestellt und eine Hämatoxylin-Eosin-Färbung (HE) und zusätzlich oder alternativ spezifische Antikörperfärbungen in an sich bekannter Weise nach Standardprotokollen durchgeführt.

Die Ergebnisse der Einbettung der PC-12 Zellen sind in den Figuren 4 und 5 dargestellt. Die eingebetteten PC-12 Zellen lassen sich im Dermisgewebe besonders durch histologische HE-Färbung sowie durch spezifischen Antikörpernachweis aufzeigen.

Die spezifische Virusreaktivierung erfolgt in einem Zeitraum von mindestens 7 Stunden bis maximal 25 Stunden vor dem Ende der Kultivierungsphase, das heißt der Fixierung der Gewebe. Zur spezifischen Virusreaktivierung wird das Gewebe UVB-Strahlung ausgesetzt. Bei einer Wellenlänge von 312 nm und einem Energieäquivalent von 1500 mJ wird jeweils für 8 Minuten bestrahlt. Die Bestrahlung wird in einem Abstand von etwa 24 Stunden wiederholt.

In einem alternativen Ansatz wird das in vitro-Testgewebe anstelle von primären Keratinozyten mit Keratinozyten aus der Zelllinie HaCaT hergestellt. Diese HaCaT-Zellen sind genetisch modifiziert in Form von knock down-Zelllinien: HaCaT/TLR2Δ und HaCaT/TLR9Δ. Mit Hilfe dieser knock down-Zelllinien kann die Rolle der jeweiligen TLR im Rahmen einer HSV-Infektion näher untersucht werden.

Zur Testung antiviraler Wirkstoffe wird eine "time- and doseresponse" Analyse durchgeführt, mit dessen Hilfe die konzentrationsabhängige Zytotoxizität sowie die antiherpetische Wirkung der einzelnen Substanz untersucht werden kann. Die Applikation der zu untersuchenden Substanz erfolgt wahlweise topikal in Pulverform oder gelöst in Airliftmedium ab Tag 0 der Airliftphase. Parallel dazu wird analog ein Kontrollansatz mit einer bekannt antiviral wirkenden Kontrollsubstanz (Aciclovir; 50 µmol/l) kultiviert.

Die anschließende Immunohistochemische Färbung mit einem für HSV1 spezifischen Antikörper zeigt die Viruslast in der mikroskopischen Untersuchung. Durch Vergleich der Färbung in dem Kontrollansatz kann eine wertende Aussage über die Wirksamkeit der konkret untersuchten Substanz getroffen werden.

## Patentansprüche

1. Mehrschichtiges biologisches in vitro-Gewebe, enthaltend:
- Dermisschicht, enthaltend eine Kollagenbiomatrix mit darin eingebetteten Fibroblasten und
- Epidermisschicht, enthaltend Epithelzellen,
**dadurch gekennzeichnet, dass** zumindest in die Dermisschicht latent viral infizierte neuronale Zellen integriert sind.

2. Gewebe nach Anspruch 1, wobei die neuronalen Zellen Phäochromocytomzellen Typ PC-12 oder davon abgeleitete Zellen sind.

3. Gewebe nach Anspruch 1 oder 2, wobei die virale Infektion eine aktivierte oder reaktivierbare latente Infektion mit Herpes simplex Virus Typ 1 (HSV-1) ist.

4. Gewebe nach einem der vorstehenden Ansprüche, wobei die Epithelzellen ausgewählt sind aus primären Keratinozyten.

5. Gewebe nach einem der vorstehenden Ansprüche, wobei die Epithelzellen ausgewählt sind aus Keratinozytenzelllinien.

6. Gewebe nach Anspruch 5, wobei die Epithelzellen die Kerationozytenzelllinie HaCaT oder davon abgeleitete Zellen sind.

7. Gewebe nach Anspruch 5 oder 6, wobei die Epithelzellen ausgewählt sind aus genetisch modifizierten Keratinozyten, die für einen oder mehrere Pattern Recognition Receptoren (PRR) defizient
sind.

8. Gewebe nach Anspruch 7, wobei der Pattern Recognition Receptor ausgewählt ist aus TLR2, TLR5 und TLR9.

9. Gewebe nach einem der vorstehenden Ansprüche, worin zumindest in der Dermisschicht zusätzlich immunkompetente Zellen integriert sind.

10. Gewebe nach Anspruch 9, wobei die immunkompetenten Zellen Langerhanszellen oder davon abgeleitete Zellen sind.

11. Gewebe nach einem der vorstehenden Ansprüche, wobei die Fibroblasten ausgewählt sind aus primären Fibroblasten.

12. Gewebe nach einem der vorstehenden Ansprüche, wobei die Kollagenbiomatrix aus nativem Kollagen Typ I besteht.

13. Verfahren zur Herstellung eines mehrschichtigen biologischen Gewebes als in vitro-Testsystem, enthaltend die Schritte
- Bereitstellen eines mehrschichtigen Gewebes, enthaltend eine Dermisschicht, enthaltend eine Kollagenbiomatrix mit darin eingebetteten Fibroblasten, und eine Epidermisschicht, enthaltend Epithelzellen und
- Aussäen von latent viral infizierten neuronalen Zellen auf das mehrschichtige biologische Gewebe, sodass diese in das biologische Gewebe integriert werden.

14. Verfahren nach Anspruch 13, wobei die neuronalen Zellen Phäocytochromzellen Typ PC-12 oder davon abgeleitete Zellen sind.

15. Verfahren nach Anspruch 13 oder 14, wobei die neuronalen Zellen durch Infektion mit Herpes Simplex Virus Typ 1 (HSV-1) vor dem Aussäen infiziert werden, um eine latente und reaktivierbare Virusinfektion zu erzeugen.

16. Verfahren zum Auffinden eines antiviral wirkenden Wirkstoffs aus einer Gruppe von zu untersuchenden Substanzen, enthaltend die Schritte:
- Bereitstellen des mehrschichtigen biologischen in vitro-Gewebes nach einem der Ansprüche 1 bis 12,
- spezifische Reaktivierung der Virusinfektion in den latent viral infizierten neuronalen Zellen des Gewebes,
- Inkontaktbringen des Gewebes, vor oder nach Virusreaktivierung, mit der zu untersuchenden Substanz,
- Nachweis des Umfangs der Virusaktivierung, wobei die Reduzierung des Umfangs der Virusaktivierung gegenüber nicht mit Substanz in Kontakt gebrachtem Gewebe eine antivirale Wirkung der Substanz anzeigt.

17. Verfahren nach Anspruch 16, wobei die Reaktivierung des Virus durch mindestens einen Stressor ausgewählt aus UVB-Exposition und Hitzeexposition, bewirkt wird.

18. Verfahren nach vorstehendem Anspruch, wobei die zu untersuchende Substanz topikal auf das biologische Gewebe aufgebracht wird.

19. Verwendung eines biologischen in vitro-Gewebes charakterisiert nach einem der Ansprüche 1 bis 12 zum Auffinden antiviral wirkender Wirkstoffe.

## Claims

1. Multi-layered biological in vitro tissue containing:
- dermis layer, containing a collagen biomatrix with fibroblasts embedded therein and
- epidermis layer, containing epithelial cells,
**characterized in that** latently virally infected neuronal cells are integrated at least into the dermis layer.

2. Tissue according to claim 1, wherein the neuronal cells are pheochromocytoma cells type PC12; or cells derived therefrom.

3. Tissue according to claim 1 or 2, wherein the viral infection is an activated or reactivatable latent infection with herpes simplex virus type 1 (HSV1).

4. Tissue according to one of the preceding claims, wherein the epithelial cells are selected from primary keratinocytes.

5. Tissue according to one of the preceding claims, wherein the epithelial cells are selected from keratinocyte cell lines.

6. Tissue according to claim 5, wherein the epithelial cells are the keratinocyte cell line HaCaT or are cells derived therefrom.

7. Tissue according to claim 5 or 6, wherein the epithelial cells are selected from genetically modified keratinocytes that are deficient for one or more pattern recognition receptors (PRR).

8. Tissue according to claim 7, wherein the pattern recognition receptor is selected from TLR2, TLR5 and TLR9.

9. Tissue according to one of the preceding claims, wherein in addition immunocompetent cells are integrated at least into the dermis layer.

10. Tissue according to claim 9, wherein the immunocompetent cells are Langerhans cells or cells derived therefrom.

11. Tissue according to one of the preceding claims, wherein the fibroblasts are selected from primary fibroblasts.

12. Tissue according to one of the preceding claims, wherein the collagen biomatrix is composed of native collagen type I.

13. Method for producing a multi-layered biological tissue as an in vitro test system, containing the steps:
- provision of a multi-layered tissue, containing a dermis layer, containing a collagen biomatrix with fibroblasts embedded therein, and an epidermis layer, containing epithelial cells and
- seeding of latently virally infected neuronal cells on the multi-layered biological tissue, so that they are integrated into the biological tissue.

14. Method according to claim 13, wherein the neuronal cells are pheochromocytoma cells type PC12, or cells derived therefrom.

15. Method according to claim 13 or 14, wherein the neuronal cells are infected before seeding by infection with herpes simplex virus type 1 (HSV1), in order to generate a latent and reactivatable virus infection.

16. Method for finding an active ingredient with an anti-viral action from a group of substances to be examined, containing the steps:
- provision of a multi-layered biological in vitro tissue according to one of claims 1 through 12,
- specific reactivation of the virus infection in the latently virally infected neuronal cells of the tissue,
- bringing the tissue before or after the virus reactivation into contact with the substance to be examined,
- verification of the extent of the virus activation, wherein the reduction of the extent of the virus activation compared to tissue not brought into contact with the substance indicates an antiviral action of the substance.

17. Method according to claim 16, wherein the reactivation of the virus is caused by at least one stressor selected from UVB exposure and heat exposure.

18. Method according to preceding claim, wherein the substance to be examined is applied topically to the biological tissue.

19. Use of a biological in vitro tissue **characterized by** one of claims 1 through 12 for finding active ingredients with an anti-viral action.

## Revendications

1. Tissu biologique in vitro multicouche contenant :
- une couche de derme contenant une biomatrice de collagène avec des fibroblastes intégrés en elle et
- une couche d'épiderme contenant des cellules épithéliales,
**caractérisé en ce que** des cellules neuronales infectées de manière virale latente sont intégrées au moins dans la couche de derme.

2. Tissu selon la revendication 1, dans lequel les cellules neuronales sont des cellules de phéochromocytome de type PC-12 ou des cellules dérivées de celles-ci.

3. Tissu selon la revendication 1 ou 2, dans lequel l'infection virale est une infection latente activée ou réactivable avec le virus de l'herpès simplex de type 1 (HSV-1).

4. Tissu selon une des revendications précédentes, dans lequel les cellules épithéliales sont sélectionnées parmi des kératinocytes primaires.

5. Tissu selon une des revendications précédentes, dans lequel les cellules épithéliales sont sélectionnées parmi des lignées cellulaires de kératinocytes.

6. Tissu selon la revendication 5, dans lequel les cellules épithéliales sont la lignée cellulaire de kératinocytes HaCaT ou des cellules dérivées de celle-ci.

7. Tissu selon la revendication 5 ou 6, dans lequel les cellules épithéliales sont sélectionnées parmi des kératinocytes génétiquement modifiés qui sont déficients en un ou plusieurs récepteurs de reconnaissance de motifs moléculaires (PRR).

8. Tissu selon la revendication 7, dans lequel le récepteur de reconnaissance de motifs moléculaires est sélectionné parmi TLR2, TLR5 et TLR9.

9. Tissu selon une des revendications précédentes, dans lequel des cellules immunocompétentes sont en outre intégrées au moins dans la couche de derme.

10. Tissu selon la revendication 9, dans lequel les cellules immunocompétentes sont des cellules de Langerhans ou des cellules dérivées de celles-ci.

11. Tissu selon une des revendications précédentes, dans lequel les fibroblastes sont sélectionnés parmi des fibroblastes primaires.

12. Tissu selon une des revendications précédentes, dans lequel la biomatrice de collagène se compose de collagène natif de type I.

13. Procédé de fabrication d'un tissu biologique multicouches en tant que système d'essai in vitro, contenant les étapes de
- préparation d'un tissu multicouches contenant une couche de derme, contenant une biomatrice de collagène avec des fibroblastes intégrés en elle, et une couche d'épiderme, contenant des cellules épithéliales et
- ensemencement de cellules neuronales infectées de manière virale latente sur le tissu biologique multicouches de sorte que celles-ci sont intégrées dans le tissu biologique.

14. Procédé selon la revendication 13, dans lequel les cellules neuronales sont des cellules de phéochromocytome de type PC-12 ou des cellules dérivées de celles-ci.

15. Procédé selon la revendication 13 ou 14, dans lequel les cellules neuronales sont infectées par infection avec le virus de l'herpès simplex de type 1 (HSV-1) avant la semence pour générer une infection virale latente et réactivable.

16. Procédé de détection d'un agent actif à action antivirale parmi un groupe de substances à examiner, contenant les étapes de :
- préparation du tissu biologique in vitro multicouche selon une des revendications 1 à 12,
- réactivation spécifique de l'infection virale dans les cellules neuronales infectées de manière virale latente du tissu,
- amenée en contact du tissu avant ou après la réactivation virale avec la substance à examiner,
- dépistage de l'étendue de l'activation virale, dans lequel la réduction de l'étendue de l'activation virale par rapport à du tissu non amené en contact avec de la substance indique une action antivirale de la substance.

17. Procédé selon la revendication 16, dans lequel la réactivation du virus est entraînée par au moins un agent stressant sélectionné parmi l'exposition aux UVB et l'exposition à la chaleur.

18. Procédé selon la revendication précédente, dans lequel la substance à examiner est appliquée de manière topique sur le tissu biologique.

19. Utilisation d'un tissu biologique in vitro caractérisé selon une des revendications 1 à 12 pour la détection d'agents actifs à action antivirale.
